# EUROPEAN PATENT APPLICATION

(11) **EP 3 492 137 A1**
(43) Date of publication of application: **05.06.2019**
(21) Application number: 17204302.8
(22) Date of filing: 29.11.2017
(51) Int. Cl.: A61N 1/36, A61N 1/05

(54) **IMPLANTABLE HEARING AID UTILIZING A STIMULATION PULSE**

(71) Applicant: Oticon Medical A/S, 2765 Smørum (DK)
(72) Inventor: STAHL, Pierre, FR-06220 Vallauris (FR); VAIARELLO, Yannick, FR-06220 Vallauris (FR)
(74) Representative: William Demant

(57) **Abstract**

A hearing aid implant includes a control unit and a stimulation unit. The control unit is configured to generate a train of biphasic electric pulses with each biphasic electric pulse comprising a stimulation pulse and a charge-compensation pulse, and to adjust a current stimulation rate to generate an adjusted stimulation rate and at least one of pulse amplitude or pulse duration of the stimulation pulse. The implant also includes a stimulation unit configured to deliver said train of electric pulses to an electrode array that is configured to provide electric stimulation according to the stimulation rate and adjusted stimulation rate. The control unit is further configured to calculate a pulse amplitude and pulse duration of the charge-compensation pulse corresponding to the stimulation pulse such that the charge-compensation pulse has the pulse amplitude and pulse duration that charge compensates the charge caused by the stimulation pulse, and the pulse duration is according to the stimulation rate and adjusted stimulation rate.

## Description

### FIELD

The disclosure relates to an implantable hearing aid configured to stimulate a cochlea utilizing biphasic electric stimulation pulses and a method of generating biphasic electric stimulation pulses.

### BACKGROUND

The sense of hearing in human beings involves the use of hair cells in the cochlea that convert or transduce acoustic signals into auditory nerve impulses. Hearing loss, which may be due to many different causes, is generally of two types: conductive and sensorineural. Conductive hearing loss occurs when the normal mechanical pathways for sound to reach the hair cells in the cochlea are impeded. These sound pathways may be impeded, for example, by damage to the auditory ossicles. Conductive hearing loss may often be overcome through the use of conventional hearing aids that amplify sound so that acoustic signals can reach the hair cells within the cochlea. Some types of conductive hearing loss may also be treated by surgical procedures.

Sensorineural hearing loss, on the other hand, is caused by the absence or destruction of the hair cells in the cochlea which are needed to transduce acoustic signals into auditory nerve impulses. People who suffer from sensorineural hearing loss may be unable to derive significant benefit from conventional hearing aid systems, no matter how intense the acoustic stimulus is. This is because the mechanism for transducing sound energy into auditory nerve impulses has been damaged. Thus, in the absence of properly functioning hair cells, auditory nerve impulses cannot be generated directly from sounds.

To overcome sensorineural hearing loss, numerous auditory prosthesis systems (e.g., cochlear implant (CI) systems) have been developed. Auditory prosthesis systems bypass the hair cells in the cochlea by presenting electrical stimulation directly to the auditory nerve fibers. Direct stimulation of the auditory nerve fibers leads to the perception of sound in the brain and at least partial restoration of hearing function.

Cochlea implant system or auditory brainstem implant system regularly comprise an external device and an internal device. In general, the external device has at least one microphone that picks up sound from the environment and generates electric audio signals representing frequency bands of the captured sound, a processing unit for processing electric audio signals and for generating a coded data stream, and a transmitter that sends power and the coded data stream across the person's skin to an internal device via electromagnetic induction. The internal device may be a hearing aid implant which typically comprises a unit to receive the coded data stream and to convert the coded data stream into a train of electric stimulation pulses. This unit could be a control unit. The system may further include an electrode array comprising a lead having an array of electrodes disposed thereon. The train of electric stimulation pulses are delivered to the electrode array which eventually provides electric stimulation pulses for electric stimulation of the cochlea nerve or the brainstem. In some implementation, the external unit device may also be implanted and the coded data stream from the implanted external unit may be transmitted to the stimulator using a wired or wireless connection.

The effectiveness of the electrical stimulation of i.e. auditory nerve fibres depends on the waveform of the electric pulses. The standard waveform used is biphasic. A biphasic pulse is a pulse pair comprising a first pulse with a first polarity and a second pulse with a second polarity opposite to the first polarity. The first pulse is a stimulation pulse having a first polarity, which is generally anodic. The stimulation pulse is intended to activate nerve fibres and, thus, have to provide stimulation above an activation threshold of i.e. auditory nerve fibres. Correspondingly, a second pulse may be a charge-compensation pulse having a second polarity, which is generally cathodic. The charge-compensation pulse compensates the charge induced by the stimulation pulse. In general, the charge of a pulse is represented by the area covered by the pulse envelope that is defined by the pulse duration and the pulse intensity over time. In case of a rectangular pulse, the charge is simply given by multiplying pulse duration and pulse amplitude of the pulse. To fulfil charge balancing, the area covered by the stimulation pulse and the area of the charge-compensation pulse of a respective biphasic electric pulse have to be same. These biphasic pulses are charge-balanced and symmetric.

It is also known that a monophasic stimulation pulse comprising only one polarity may also be used for electrical stimulation and requires less current for stimulating the auditory nerve fibres, thus is more efficient than the symmetric biphasic stimulation pulses. However, monophasic stimulation pulses are not used because charge-unbalanced pulses induce faradaic chemical reactions that may damage biological tissue.

The disclosure provides an alternative to the stimulation pulses that are conventionally used for electrical stimulation to generate hearing sensation.

### SUMMARY

According to an embodiment, a hearing aid implant is disclosed. The hearing aid implant includes a control unit and a stimulator unit. The control unit may be configured to generate a train of biphasic electric pulses in dependence upon an electric audio signal, wherein each biphasic electric pulse comprises a stimulation pulse of a first polarity and a charge-compensation pulse of a second polarity opposite to said first polarity of the stimulation pulse. The control unit may be configured to adjust a current stimulation rate relating to a first stimulation pulse of said train of biphasic electric pulses to generate an adjusted stimulation rate relating to a second stimulation pulse of said train of biphasic electric pulses. The control unit may further be configured to adjust at least one of pulse amplitude or pulse duration relating to the first stimulation pulse and at least one of pulse amplitude or pulse duration relating to the second stimulation pulse. The stimulation unit may be configured to deliver said train of electric pulses to an electrode array, said electrode array comprising a number of electrodes for providing electric stimulation with the first stimulation pulse at the current stimulation rate and/ or with the second stimulation pulse at the adjusted stimulation rate. The control unit may further be configured to i) calculate a first pulse amplitude and first pulse duration of a first charge-compensation pulse corresponding to the first stimulation pulse such that the first charge-compensation pulse has the first pulse amplitude and first pulse duration that result in a charge compensation of the charge caused by the first stimulation pulse, and the first pulse duration is according to the current stimulation rate, and/ or ii) calculate a second pulse amplitude and second pulse duration of a second charge-compensation pulse corresponding to the second stimulation pulse such that the charge-compensation pulse has the second pulse amplitude and second pulse duration that result in a charge compensation of the charge caused by the second stimulation pulse, and the second pulse duration is according to the adjusted stimulation rate.

The functionality of the control unit and the stimulation unit may be comprised in a single unit such as an implantable processor of the hearing aid implant.

The stimulation rate may be defined as a rate at which one cycle of stimulation occurs, i.e. inverse of duration of one cycle of stimulation. In other words, the duration of one cycle of stimulation includes the period of time between onset time point of a first pulse of a biphasic pulse and the onset time point of a first pulse of a consecutive biphasic pulse. Such stimulation would include stimulation utilizing one electric biphasic pulse comprising delivery of the stimulation pulse followed by charge compensation as through the charge compensation pulse. The stimulation pulse may be anodic, in which case the charge compensation pulse is cathodic. Alternatively, the stimulation pulse may be cathodic and the charge compensation pulse is accordingly anodic. The stimulation rate may also take into account any delay, for example between an end time point of the stimulation pulse and onset time point of the charge compensation pulse and/ or between an end time point of a charge compensation pulse and onset time point of following stimulation pulse. Generally, the stimulation rate may be adjusted during operation of a hearing aid implant. This may include the control unit adjusting a current stimulation rate relating to a first electric biphasic pulse to generate an adjusted stimulation rate that may relate to a following second electric biphasic pulse. Thus, the rate at which two consecutive biphasic pulses travel may be varied pulse-to-pulse. Or in other words, the current period duration that is the inverse of the current stimulation rate might vary pulse-to-pulse. A current period duration can for example be derived from the time span between two consecutives stimulation pulses of two respective biphasic pulses.

The stimulation rate may be periodic or aperiodic. The control unit may be configured to adjust the stimulation rate according to the hearing situation or according to a user's hearing impairment.

In an embodiment, the stimulation pulse and charge-compensation pulse for at least one of the electric biphasic pulse of the train of biphasic pulses are individually rectangular and asymmetric to each other, wherein the control unit is configured to calculate a ratio specific to the stimulation rate of the at least one of the electric biphasic pulse and the asymmetry is defined by the calculated ratio. The ratio is defined as a ratio between a pulse duration of the stimulation pulse and pulse duration of the charge-compensation pulse. The asymmetry refers to the stimulation pulse and charge compensation pulse not being identical to each other. For example, the stimulation pulse with a first polarity has a pulse duration and the charge compensation pulse with the second polarity opposite to the first polarity of the first pulse has a second pulse duration that is larger than the pulse duration of the stimulation pulse. With the prerequisite of charge-balancing it becomes immediately clear, that if the pulse duration of the stimulation pulse is shorter than the pulse duration of the charge compensation pulse, the pulse amplitude of the stimulation pulse is higher than the pulse amplitude of the charge-compensation pulse. Thus, if the pulse duration of the charge compensation pulse is minimized the waveform of the asymmetric biphasic pulse tends to represent a waveform of a (pseudo-) monophasic pulse. It is identified that, a stimulation efficiency of a pseudo-monophasic pulse increases with increasing pulse duration or synonymously with decreasing pulse amplitude of the charge compensation pulse.

In an embodiment, the first pulse duration corresponding to the current stimulation rate is at least a part of a first remaining time. The control unit is configured to determine the first remaining time by calculating a difference between inverse of the current stimulation rate and the pulse duration relating to the first stimulation pulse or between inverse of the current stimulation rate and the pulse duration relating to the first stimulation pulse and at least one of a delay defined by a duration between an end time point of the first stimulation pulse and an onset time point of the first charge-compensation pulse or a delay defined by a duration between an onset time point of a first biphasic pulse including the first stimulation pulse and an onset time point of the first stimulation pulse of the first biphasic pulse. Additionally or alternatively, the second pulse duration corresponding to the adjusted stimulation rate is at least a part of a second remaining time. The control unit is further configured to determine the second remaining time by calculating a difference between inverse of the adjusted stimulation rate and the pulse duration relating to the second stimulation pulse or between inverse of the adjusted stimulation rate and the pulse duration relating to the second stimulation pulse and at least one of a delay defined by a duration between an end time point of the second stimulation pulse and an onset time point of the second charge-compensation pulse or a delay defined by a duration between an onset time point of a second biphasic pulse including the second stimulation pulse and an onset time point of the second stimulation pulse of the second biphasic pulse. In these embodiments, the control unit is configured to initially calculate the inverse of the stimulation rate and/ or adjusted stimulation rate.

In an embodiment, the control unit is configured to maximize the first pulse duration by utilizing the first remaining time entirely as the first pulse duration and accordingly calculate the first pulse amplitude to charge compensate the charge caused by the first stimulation pulse. Additionally or alternatively, the control unit is further configured to maximize the second pulse duration utilizing the second remaining time entirely as the second pulse duration and accordingly calculate the second pulse amplitude to charge compensate the charge caused by the second stimulation pulse. With an increase in pulse duration or synonymously decrease in pulse amplitude of the charge compensation pulse, this approach allows for increasing the stimulation efficiency.

The waveform of the biphasic pulse may be characterized by a ratio between the pulse amplitude of the stimulation pulse and the pulse amplitude of the charge-compensation pulse. An equivalent definition of the ratio may be formulated using the pulse duration of the stimulation pulse and the pulse duration of the charge-compensation pulse. The disclosure allows for providing a train of biphasic pulses where the ratio may be made non-fixed, i.e. variable across different biphasic pulses in the train of biphasic pulses. This avoids the fixed ratio approach that is generally utilized in conventionally known auditory hearing systems like cochlear implants.

It was recognized that conventionally used fixed ratio limits efficiency of stimulation and is thus, incompatible with high stimulation rates required in cochlea or brainstem implants. However, the disclosed variable ratio approach overcomes this problem and allows for utilizing high stimulation rates. It was also recognized that a variable ratio allows for providing an effectiveness as close as possible to a monophasic stimulation pulse with the prerequisite of charge-balancing in order to prevent damaging of tissue.

Considering an exemplified scenario that the current stimulation rate is lower than the highest stimulation rate for the train of electric biphasic pulses or if the pulse duration of the stimulation pulse is shorter than the longest pulse duration of the stimulation pulse for the train of electric biphasic pulses, the waveform may be individually adjusted for at least one, preferably each, of the biphasic pulses. Consequently, the efficiency of an adjusted pulse would be improved compared to the waveform used in prior art solutions, which typically relies on using a fixed ratio across the biphasic pulses.

In an embodiment, the control unit is configured to vary a ratio between a pulse amplitude of a stimulation pulse and pulse amplitude of corresponding charge-compensation pulse across different biphasic pulses of the train of biphasic pulses.

In another embodiment, the control unit is configured to vary a ratio between a pulse amplitude of the stimulation pulse and pulse amplitude of corresponding charge-compensation pulse of at least one biphasic electric pulse during hearing aid implant operation in accordance with a ratio between the pulse duration of the charge-compensation pulse and the pulse duration of the stimulation pulse of the at least one biphasic electric pulse.

In another embodiment, the control unit is configured to calculate a first ratio as a ratio between a pulse duration of a first stimulation pulse and first pulse duration of a first charge compensation pulse of a first biphasic pulse and calculate a second ratio as a ratio between a pulse duration of a second stimulation pulse and a second pulse duration of a second charge compensation pulse of a second biphasic pulse. The control unit is further configured to change the first ratio for the first biphasic pulse comprising the first stimulation pulse and first charge-compensation pulse to the second ratio for the second biphasic pulse comprising the second stimulation pulse and second charge-compensation pulse. As it is apparent, the first ratio is also representative of a ratio between the pulse amplitude of the first stimulation pulse and first charge-compensation pulse and the second ratio is representative of a ratio between the pulse amplitude of the second stimulation pulse and second charge-compensation pulse.

Accordingly, the control unit may be configured to adjust the pulse amplitude and pulse duration of at least one charge-compensation pulse of a respective biphasic electric pulse such that the charge-compensation pulse has a pulse amplitude and a pulse duration that compensate the charge caused by the stimulation pulse, wherein the pulse duration of said charge-compensation pulse is modified according to the stimulation rate relating to the respective biphasic electric pulse.

In conventional cochlear implant system or auditory brainstem implant system, sound is picked up by a microphone or microphone array and converted to electric audio signals which represent frequency bands of the captured sound. Typically, an external processor processes the band limited electric audio signals and transmits the processed electric audio signals as a coded data stream over an inductive link to the internal device, the implant. The implant comprises a control unit that decodes the received data and based on the decoded data generates a train of biphasic pulses in dependence on the electric audio signal and a stimulator unit that is configured to deliver the train of biphasic pulses to an implantable electrode array.

In an embodiment, the external sound processor and/ or microphone may also be implanted. If a cochlea or brainstem implant comprises an implanted device, only, the implanted device could comprise a microphone to pick up sound and to convert the captured sound into electric audio signals. An internal processor then processes the band limited electric audio signal which will be provided as a coded data stream to the control unit. The control unit generates a train of biphasic electric pulses in dependence on an electric audio signal and the stimulator unit delivers the train of biphasic electric pulses to an implantable electrode array. In this case no transmission of the coded data over an inductive link is necessary.

The biphasic electric pulses comprise a stimulation pulse of a first polarity and a charge-compensation pulse of a second polarity opposite to the first polarity of the stimulation pulse. The stimulation pulse is intended to provide electrical stimulation above a stimulation threshold of i.e. auditory nerve fibres and the charge-compensation pulse is intended to compensate the charge induced by the stimulation pulse. The first polarity may be anodic and the second polarity cathodic or vice versa. The stimulation threshold is frequency dependent and based on the threshold level (T-level), which may be ascertained using known technique such as evoked action compound potential (ECAP) measurements.

The fact, that the control unit is configured to adjust the pulse duration of the charge-compensation pulse according to the stimulation rate of a specific biphasic pulse of the train of biphasic electric pulses leads to the consequence that the ratio between the pulse amplitude of the stimulation pulse and pulse amplitude of the charge-compensation pulse is may vary, i.e. not fixed. Hence, each biphasic pulse can be adjusted individually according to the respective stimulation rate and optimized with respect to the stimulation efficiency. The described process is based on the requirements of charge-balancing and the idea of using the full time available between two consecutive biphasic pulses to extend the pulse duration of the charge-compensation pulse preferably over a maximum remaining time. As a result, the pulse amplitude of the respective charge-compensation pulse is minimized and the efficiency of the biphasic pulse is maximized.

The hearing aid implant may be implemented for an anodic or cathodic stimulation pulse and accordingly, the charge-compensation pulse may be cathodic or anodic respectively. In these scenarios, the cathodic pulse may be adjusted according to the stimulation rate of respective biphasic pulse and pulse amplitude and duration of the anodic pulse of the respective biphasic pulse. Variants of the hearing aid implant may implement single, sequential or multi-electrode stimulation.

In a preferred embodiment, the control unit is further configured to calculate the pulse duration of said charge-compensation pulse based on at least one of the current period duration that is the inverse of the current stimulation rate, a time period for which different current sources, voltage sinks are occupied, the pulse amplitude and/or the pulse duration of said stimulation pulse and/or a number of current sources and/or voltage sinks available.

One preferred way of defining the current period duration is to take the time between the onset time point of a first pulse of a biphasic pulse and the onset time point of a first pulse of a consecutive biphasic pulse. Using this definition, one can also define a time window between two consecutive biphasic pulses. The time window extends from the end time point of the second pulse of the first biphasic pulse to the onset time point of the first pulse of a consecutive biphasic pulse. By subtracting the pulse duration of a first pulse of the first biphasic pulse from the current period duration between the first biphasic pulse and the consecutive pulse, the maximum time widow available can be derived. The maximum time window available provides the maximum possible pulse duration over which the charge-compensation pulse of a first biphasic pulse can extend.

An alternative way of calculating the pulse duration of a charge-compensation pulse is provided by the time period for which different current sources and/or voltage sinks are occupied. The terms source and sink may refer to the same object in dependence of the perspective of an observer. Hereby, current theoretically in formalism or physically in a real system enters or exits a system which can be used to derive the current period duration. Alternatively, the number of current sources and/or voltage sinks available can be used, too. To calculate the pulse duration of the charge-compensation pulse also pulse amplitude and pulse duration of the stimulation pulse of the respective biphasic pulse can be taken into account separately or in combination with one or more of the other methods.

In an alternative embodiment that may be combined with the previous embodiment calculating the pulse duration of said charge-compensation pulse includes subtracting the pulse duration of said stimulation pulse or pulse duration of said stimulation pulse and a delay defined by a duration between an end time point of the stimulation pulse and an onset time point of the charge-compensation pulse from said current period duration.

In an embodiment, by subtracting the pulse duration of a stimulation pulse from the current period duration a maximum time window available between a stimulation pulse of a biphasic pulse and a stimulation pulse of a consecutive biphasic pulse can be derived. If there is no delay between the end time point of the stimulation pulse of the first biphasic and the onset time point of the charge-compensation pulse of the first biphasic pulse and if there is no delay between the end time point of said charge-compensation pulse and the onset of the stimulation pulse of the consecutive biphasic pulse, the time window directly corresponds to the maximum possible pulse duration of the respective charge-compensation pulse. However, if there exists a delay, i.e. between the end time point of the stimulation pulse of the first biphasic pulse and the onset time point of said charge-compensation pulse and/or a delay between the end time point of the charge-compensation pulse and the onset time point of the stimulation pulse of the consecutive biphasic pulse, the delay should be taken into account for calculating the pulse duration of the charge-compensation pulse.

In a preferred embodiment, the control unit is further configured to calculate the pulse duration of said charge-compensation pulse by subtracting the pulse duration of said stimulation pulse or the pulse duration of said stimulation pulse and a delay defined by a duration between an end time point of the stimulation pulse and an onset time point of the charge-compensation pulse from a current period duration, the control unit being configured to calculate the current period duration as an inverse of the current stimulation rate.

In an emdbodiment, the control unit is configured to initially calculate the current period duration that is an inverse of the current stimulation rate. Using the current period duration the control unit calculates the pulse duration of the respective charge-compensation pulseb of a first biphasic pulse by subtracting either the pulse duration of the stimulation pulse of the first biphasic pulse or by subtracting the pulse duration of the stimulation pulse and a delay defined by a duration between an end time point of the stimulation pulse and an onset time point of the charge-compensation pulse from a current period duration. Alternatively, if there exists a delay between an end time point of the charge-compensation pulse and an onset time point of the stimulation pulse of the consecutive biphasic pulse, this also can be taken into account for calculating the pulse duration of the charge-compensation pulse. The delay can occur between an end time point of the stimulation pulse and an onset time point of the charge-compensation pulse and/or between an end time point of the charge-compensation pulse and an onset time point of the stimulation pulse of the consecutive biphasic pulse.

The fact, that the pulse duration of the charge-compensation pulse can be adjusted according to the stimulation rate leads to the consequence that the ratio between the pulse amplitudes of the stimulation pulse and pulse amplitude of the charge-compensation pulse of at least one biphasic electric pulse can vary compared to the ratio at other biphasic pulses of the train of biphasic pulses. In an extreme case, the ratio between the pulse amplitudes of the stimulation pulse and pulse amplitude of the charge-compensation pulse of each biphasic electric pulse within a train of biphasic pulses is different from the ratio of other biphasic electric pulses. The control unit may be configured to adjust the stimulation rate for example according a changing hearing situation during hearing aid implant operation. Consequently, the stimulation rate may vary during hearing aid implant operation. A variation of the stimulation rate may be a possible reason why the ratio between the pulse amplitude of the stimulation pulse and pulse amplitude of the charge-compensation pulse of at least one biphasic electric pulse may vary during hearing aid implant operation. Also varying amplitudes of the stimulation pulses can lead to varying ratios within a train of biphasic electric pulses.

As to the definition of a ratio as the ratio between the pulse amplitude of the stimulation pulse and pulse amplitude of the charge-compensation pulse a ratio can also be defined as the ratio between the pulse duration of the charge-compensation pulse and pulse duration of the stimulation pulse of at least one biphasic electric pulse. In view of charge balancing, this definition is equivalent to the definition of a ratio as the ratio between the pulse amplitudes of the stimulation pulse and pulse amplitude of the charge-compensation pulse.

In another preferred embodiment, the control unit is configured to maximize the pulse duration and to minimize the pulse amplitude of at least one charge-compensation pulse during hearing aid implant operation. Maximizing the pulse duration may refer to a process in which the pulse duration of the charge-compensation pulse tends to the case of using the maximum possible time window available. Accordingly, in view of the prerequisite of charge-balancing, when the pulse duration of the charge-compensation pulse is maximized the pulse amplitude of the charge-compensation pulse is minimized. In case the pulse duration of the charge-compensation pulse is at its maximum, the pulse amplitude of the charge-compensation pulse is at its minimum. This situation states the most efficient waveform of a respective biphasic pulse as this waveform is the closest to the pure monophasic waveform. Apparently, the waveform of a biphasic pulse having maximum pulse duration and minimum amplitude of the respective charge-compensation pulse is limited by the boundary conditions of the pulse amplitude and the pulse duration of the respective stimulation pulse of the biphasic pulse and the stimulation rate.

In general one may state that the higher the ratio either between the pulse amplitude of the stimulation pulse and pulse amplitude of the charge-compensation pulse or between the pulse duration of the charge-compensation pulse and pulse duration of the stimulation pulse of at least one biphasic electric pulse the closer is the biphasic waveform to a pure monophasic waveform and, thus, the more efficient is the stimulation with the respective biphasic electric pulse.

An efficient stimulation may be defined as reduction in power consumption. A further advantage of using the biphasic pulse, as optimized according to the disclosure, includes reduced channel interactions, which are associated with poorer speech understanding.

In another embodiment, the control unit is configured to adjust pulse amplitude and pulse duration of at least one charge-compensation pulse for providing single and/or sequential and/or multi electrode stimulation.

In an embodiment, the electrode array is configured to be positioned within a cochlea and/or within a modiolus and/ or at an auditory brainstem of a user of the hearing aid implant. The hearing aid implant may for example be an internal device of a cochlea implant or an auditory brainstem implant or a transmdiolar implant system that can be implanted and that receives a coded data stream via an inductive link from an external device. If the hearing aid implant is comprised in a cochlea implant the electrode array provides a train of biphasic electric pulses for stimulating the cochlea nerve. In case that the hearing aid implant is comprised in an auditory brainstem implant the electrode array provides a train of biphasic electric pulses for stimulating a person's brainstem. In case that the hearing aid implant is comprised in a transmdiolar implant system, the electrode array is implant in the modiolus and directly provides a train of biphasic electric pulses to the auditory nerve. These alternatives intend to bypass a person's normal hearing process.

The hearing aid implant of at least one of the preceding embodiments may also be comprised in an implantable hearing aid system. The hearing aid system can be selected from a group consisting of cochlear implant, auditory brainstem implant and transmodiolar implant that comprises an electrode array configured to be positioned within the modiolus of a user.

Thus, in an embodiment, an implantable hearing aid system is disclosed. The hearing aid system is selected from a group consisting of cochlear implant, auditory brainstem implant and transmodiolar implant that comprises an electrode array configured to be positioned within the modiolus of a user. The implantable hearing aid system is configured to include one or more features that are previously disclosed.

According to another aspect, a method is disclosed. The method includes generating a train of biphasic electric pulses in dependence upon an electric audio signal is disclosed, wherein each biphasic electric pulse comprises a stimulation pulse of a first polarity and a charge-compensation pulse of a second polarity that is opposite to said first polarity of the stimulation pulse. The method further includes adjusting a current stimulation rate relating to a first stimulation pulse of said train of biphasic electric pulses to generate an adjusted stimulation rate relating to a second stimulation pulse of said train of biphasic electric pulses and adjusting at least one of pulse amplitude or pulse duration relating to the first stimulation pulse and at least one of pulse amplitude or pulse duration relating to the second stimulation pulse. The method further includes delivering a first biphasic electric pulse and/ or a second biphasic electric pulse of the train of biphasic electric pulses to an electrode array comprising a number of electrodes, the first biphasic electric pulse comprising the first stimulation pulse and a first change-compensation electric pulse and the second biphasic electric pulse comprising the second stimulation pulse and a second charge compensation electric pulse; calculating a first pulse amplitude and first pulse duration of the first charge-compensation pulse such that the first charge-compensation pulse has the first pulse amplitude and first pulse duration that result in a charge compensation of the charge caused by the first stimulation pulse, and the first pulse duration is according to the current stimulation rate, and/ or calculating a second pulse amplitude and second pulse duration of the second charge-compensation pulse such that the charge-compensation pulse has the second pulse amplitude and second pulse duration that result in a charge compensation of the charge caused by the second stimulation pulse, and the second pulse duration is according to the adjusted stimulation rate.

According to an embodiment, the method further includes calculating a difference between i) an inverse of the current stimulation rate, and ii) the pulse duration relating to the first stimulation pulse or the pulse duration relating to the first stimulation pulse and a delay, the delay being defined by a duration between an end time point of the first stimulation pulse and an onset time point of the first charge-compensation pulse, and determining the first pulse duration comprising at least a part of the calculate difference. Additionally or alternatively, the method further includes determining the second pulse duration by calculating a difference between i) an inverse of the adjusted stimulation rate, and ii) the pulse duration relating to the second stimulation pulse or the pulse duration relating to the second stimulation pulse and a delay, the delay being defined by a duration between an end time point of the second stimulation pulse and an onset time point of the second charge-compensation pulse, and determining the first pulse duration comprising at least a part of the calculate difference.

In an embodiment, the method further includes calculating the first pulse amplitude to charge compensate the charge caused by the first stimulation pulse based on the determined first pulse duration. Additionally or alternatively, the method further includes calculating the second pulse amplitude to charge compensate the charge caused by the second stimulation pulse based on the determined second pulse duration.

In an embodiment, the method includes varying a ratio between a pulse amplitude of the stimulation pulse and pulse amplitude of corresponding charge-compensation pulse of at least one biphasic electric pulse during hearing aid implant operation in accordance with a ratio between the pulse duration of the charge-compensation pulse and the pulse duration of the stimulation pulse of the at least one biphasic electric pulse.

According to an embodiment, the method includes calculating a first ratio as a ratio between a pulse duration of a first stimulation pulse and first pulse duration of a first charge compensation pulse of a first biphasic pulse and calculating a second ratio as a ratio between a pulse duration of a second stimulation pulse and a second pulse duration of a second charge compensation pulse of a second biphasic pulse; and changing the first ratio for the first biphasic pulse to the second ratio for the second biphasic pulse.

The steps of the method of at last one of the preceding variants can be performed by a data processing system comprising a processor and program code means. The program code can be adapted to cause the processor to perform the steps of the method of at last one of the preceding variants. The processing system can be part of the hearing at implant. It can for example be comprised in the control unit. In an alternative embodiment it can include both the control unit and the stimulation unit.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- Fig. 1:: shows a sketch of a hearing aid comprising an external device and a hearing aid implant according to an embodiment;
- Fig. 2A:: shows conventionally known monophasic electric pulses according to prior art;
- Fig. 2B:: shows conventionally known biphasic electric pulses according to prior art;
- Fig. 2C:: shows asymmetric biphasic electric pulses according to prior art;
- Fig. 3:: shows the implementation of an a train of asymmetric biphasic pulse according to prior art;
- Fig. 4:: shows the implementation of an pseudo-monophasic pulse according to an embodiment.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details.

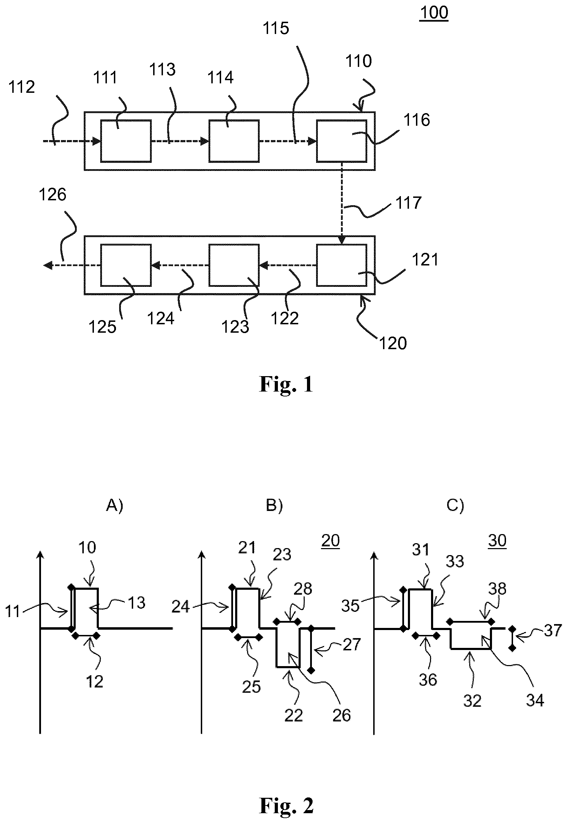

Fig. 1 shows a hearing aid system 100 comprising an external device 110 and an internal device, i.e. hearing aid implant 120 according to an embodiment. The hearing aid system 100 can be i.e. a cochlea implant or an auditory brainstem implant or transmodiolar implant. The external device 110 has a microphone 110 that is configured to pick up sound 112 from a user's environment and generates electric audio signals 113 representing frequency bands of the captured sound 112. The external device 110 further comprises a processing unit 114 for processing electric audio signals 113 and for generating a coded data stream 115. The coded data stream 115 is delivered to a transmitter 116 that sends power and the coded data stream 115 across the person's skin to an internal device 120 via electromagnetic induction 117. The internal device 120 is a hearing aid implant which is implanted into a user's body to provide electric stimulation for example to auditory nerve fibres. In the embodiment shown, the internal device 120 comprises a control unit 121 that receives the coded data stream 115 and converts the coded data stream 115 into a train of electric pulses 122. Through a further comprised stimulation unit 123, the train of electric pulses 122 is delivered 124 to an electrode array 125 which eventually provides stimulation pulses 126 for electric stimulation of i.e. the auditory nerve or the brainstem at a given stimulation rate.

In an alternative to the embodiment shown in Fig. 1, a microphone and a processing unit are comprised in an internal device that is implanted into a user's body. In this alternative embodiment no transmitter needs to be integrated. In a further alternative embodiment, a stimulation unit is integrated into a control unit.

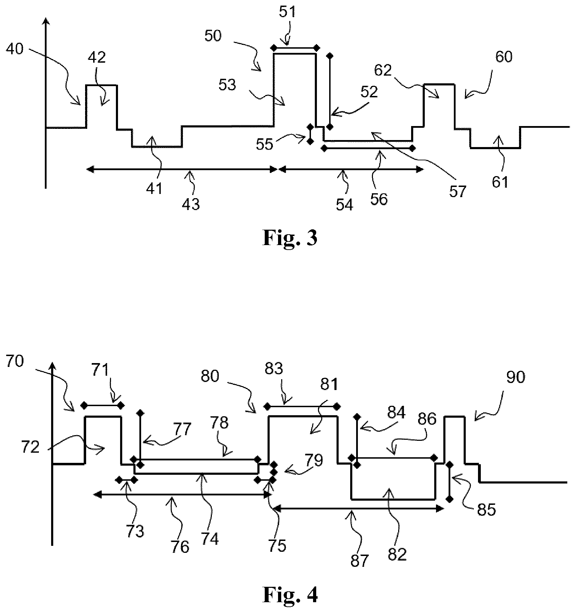

In an embodiment, the control unit 121 is configured to generate a train of biphasic electric pulses (Fig. 4, 72, 74, 81, 82) in dependence upon an electric audio signal 113, wherein each biphasic electric pulse comprises a stimulation pulse (Fig. 4, 72, 81) of a first polarity and a charge-compensation pulse (74, 82) of a second polarity opposite to said first polarity of the stimulation pulse. The control unit 121 is further configured to i) adjust a current stimulation rate (inverse of duration 76, Fig. 4) relating to a first stimulation pulse (Fig. 4, 72) of said train of biphasic electric pulses to generate an adjusted stimulation rate (inverse of duration 87, Fig. 4) relating to a second stimulation pulse (fig. 4, 81) of said train of biphasic electric pulses, and ii) adjust at least one of pulse amplitude (Fig. 4, 77) or pulse duration (Fig. 4, 71) relating to the first stimulation pulse (Fig. 4, 72) and at least one of pulse amplitude (Fig. 4, 84) or pulse duration (Fig. 4, 83) relating to the second stimulation pulse ((Fig. 4, 81). The stimulation unit 123 is configured to deliver said train of biphasic electric pulses to an electrode array 125, said electrode array comprising a number of electrodes for providing electric stimulation with the first stimulation pulse (Fig. 4, 72) at the current stimulation rate (inverse of duration 76, Fig. 4) and/ or with the second stimulation pulse (Fig. 4, 81) at the adjusted stimulation rate (inverse of duration 87, Fig. 4). The control unit 121 is further configured to calculate a first pulse amplitude (Fig. 4, 79) and first pulse duration (Fig. 4, 78) of a first charge-compensation pulse (Fig. 4, 74) corresponding to the first stimulation pulse (Fig. 4, 72) such that the first charge-compensation pulse has the first pulse amplitude (Fig. 4, 74) and first pulse duration (Fig. 4, 78) that result in a charge compensation of the charge caused by the first stimulation pulse (Fig. 4, 72), and the first pulse duration (Fig. 4, 78) is according to the current stimulation rate (inverse of duration 76, Fig. 4), and/ or calculate a second pulse amplitude (Fig. 4, 85) and second pulse duration (Fig. 4, 86) of a second charge-compensation pulse (Fig. 4, 82) corresponding to the second stimulation pulse (Fig. 4, 81) such that the charge-compensation pulse (Fig. 4, 82) has the second pulse amplitude (Fig. 4, 85) and second pulse duration (Fig. 4, 86) that result in a charge compensation of the charge caused by the second stimulation pulse (Fig. 4, 81), and the second pulse duration is according to the adjusted stimulation rate (inverse of duration 87, Fig. 4).

According to an embodiment, the first pulse duration (Fig. 4, 78) corresponding to the current stimulation rate (inverse of duration 76, Fig. 4) is at least a part of a first remaining time (Fig. 4, 78), which the control unit 121 is configured to determine by calculating a difference between inverse of the current stimulation rate (duration 76, Fig. 4)) and the pulse duration (Fig. 4, 71) relating to the first stimulation pulse (Fig. 4, 72) or between inverse of the current stimulation rate (duration 76, Fig. 4) and the pulse duration (Fig. 4, 71) relating to the first stimulation pulse (Fig. 4, 72) and at least one of a delay (Fig. 4, 73) defined by a duration between an end time point of the first stimulation pulse (Fig. 4, 72) and an onset time point of the first charge-compensation pulse (Fig. 4, 74) or a delay defined by a duration between an onset time point of a first biphasic pulse (Fig. 4, 70) including the first stimulation pulse (Fig.4, 72) and an onset time point of the first stimulation pulse (Fig.4, 72) of the first biphasic pulse (Fig. 4, 70). Additionally or alternatively, the second pulse duration (Fig. 4, 86) corresponding to the adjusted stimulation rate (duration 87, Fig. 4) is at least a part of a second remaining time (Fig. 4, 86), which the control unit 121 is configured to determine by calculating a difference between inverse of the adjusted stimulation rate (duration 87, Fig. 4) and the pulse duration (Fig. 4, 83) relating to the second stimulation pulse (Fig. 4, 81) or between inverse of the adjusted stimulation rate (duration 87, Fig. 4) and the pulse duration (Fig. 4, 83) relating to the second stimulation pulse (Fig. 4, 81) and at least one of a delay defined by a duration between an end time point of the second stimulation pulse and an onset time point of the second charge-compensation pulse or a delay (Fig.4, 75) defined by a duration between an onset time point of a second biphasic pulse (Fig. 4, 80) including the second stimulation pulse (Fig.4, 81) and an onset time point of the second stimulation pulse (Fig.4, 81) of the second biphasic pulse (Fig. 4, 80).

According to an embodiment, the control unit 121 is configured to maximize the first pulse duration (Fig.4, 78) by utilizing the first remaining time entirely as the first pulse duration (Fig.4, 78) and accordingly calculate the first pulse amplitude (Fig.4, 79) to charge compensate the charge caused by the first stimulation pulse (Fig.4, 72). Additionally or alternatively, the control unit 121 is further configured to maximize the second pulse duration (Fig.4, 86) utilizing the second remaining time entirely as the second pulse duration (Fig.4, 86) and accordingly calculate the second pulse amplitude (Fig.4, 85) to charge compensate the charge caused by the second stimulation pulse (Fig.4, 81).

According to an embodiment, the stimulation pulse (Fig.4, 72, 81) and charge-compensation pulse (Fig.4, 74, 82) for at least one of the electric biphasic pulse (Fig. 4, 70, 80, 90) of the train of biphasic pulses (Fig. 4, 70, 80, 90) are individually rectangular and asymmetric to each other, wherein the control unit 121 is configured to calculate a ratio (Fig. 4, duration 74/ duration 71, or duration 86/ duration 83) specific to the stimulation rate (Fig. 4, inverse of duration 76, inverse of duration 87) of the at least one of the electric biphasic pulse and the asymmetry is defined by the calculated ratio.

According to an embodiment, the control unit is configured to vary a ratio (Fig. 4, amplitude 77/ amplitude 79, or amplitude 84/ amplitude 85) between a pulse amplitude (Fig. 4, 77, 84) of a stimulation pulse (Fig. 4, 72, 81) and pulse amplitude (Fig. 4, 79, 85) of corresponding charge-compensation pulse (Fig. 4, 74, 82) across different biphasic pulses (Fig. 4, 70, 80, 90) of the train of biphasic pulses (Fig. 4, 70, 80, 90).

According to an embodiment, the control unit 121 is configured to vary a ratio (Fig. 4, amplitude 77/ amplitude 79, or amplitude 84/ amplitude 85) between a pulse amplitude (Fig. 4, 77, 84) of the stimulation pulse (Fig. 4, 72, 81) and pulse amplitude (Fig. 4, 79, 85) of corresponding charge-compensation pulse (Fig. 4, 74, 82) of at least one biphasic electric pulse (Fig. 4, 70, 80) during hearing aid implant 120 operation in accordance with a ratio (Fig. 4, duration 74/ duration 71, or duration 86/ duration 83) between the pulse duration (Fig. 4, 78, 86) of the charge-compensation pulse (Fig.4, 74, 82) and the pulse duration (Fig. 4, 71, 83) of the stimulation pulse (Fig. 4, 72, 81) of the at least one biphasic electric pulse (Fig. 4, 70, 80).

According to an embodiment, the control unit 121 is configured to calculate a first ratio (Fig. 4, duration 74/ duration 71) as a ratio between a pulse duration (Fig. 71) of a first stimulation pulse (Fig. 4, 71) and first pulse duration (Fig. 4, 78) of a first charge compensation pulse (Fig. 74) of a first biphasic pulse (Fig. 4, 70) and calculate a second ratio (Fig.4, duration 86/ duration 83) as a ratio between a pulse duration (Fig. 4, 83) of a second stimulation pulse (Fig. 4, 81) and a second pulse duration (Fig. 4, 86) of a second charge compensation pulse (82) of a second biphasic pulse (Fig. 4, 80); and change the first ratio for the first biphasic pulse (Fig. 4, 70) comprising the first stimulation pulse (Fig. 4, 72) and first charge-compensation pulse (Fig. 4, 74) to the second ratio for the second biphasic pulse (Fig. 4, 80) comprising the second stimulation pulse (Fig. 4, 81) and second charge-compensation pulse (Fig.4, 82).

Figs. 2, 3 and 4 are time-intensity diagrams illustrating electric pulses by way of duration and amplitude. The amplitude can be measured in terms of current or voltage. In Figs. 2, 3 and 4, the waveforms are shown as being rectangular. However, in reality, the amplitude of each pulse may decay during pulse duration.

Fig. 2A) shows an anodic monophasic pulse 10 as proposed in the prior art. The charge induced by the monophasic pulse is represented by the area 13 covered by the pulse in a time-intesity diagram as shown in figure 2. In the embodiment of figure 2A), the pulse shape is rectangular such that the area 13 covered by the pulse is obtained by multiplying the pulse amplitude 11 by the pulse duration 12. The monophasic pulse is not charge-balanced and, thus, may induce faradaic chemical reactions that damage biological tissue. Nevertheless, this waveform is the most efficient in terms of stimulation efficiency.

Figure 2B) shows a biphasic pulse 20 comprising an anodic pulse 21 and a cathodic pulse 22 as used in the prior art. The anodic pulse 21 is the stimulation pulse and the cathodic pulse 22 is the charge-compensation pulse. The waveforms of the anodic pulse 21 and the cathodic pulse 22 are rectangular. The area 23 covered by the anodic pulse 21 is defined by the pulse amplitude 24 and the pulse duration 25 and corresponds to the charge induced by the anodic pulse. The area 26 covered by the cathodic pulse 22 is defined by the pulse amplitude 27 and the pulse duration 28 and corresponds to the charge induced by the cathodic pulse 22. Here, the absolute values of the charges induced by the anodic pulse 21 and the cathodic pulse 22 are identical such that the biphasic pulse is charge-balanced. Moreover, the absolute values of pulse amplitude 24 and pulse duration 25 of the anodic pulse 21 and pulse amplitude 27 and pulse duration 28 of the cathodic pulse 22 are identical such that the waveform of the biphasic pulse 20 is also symmetric.

Figure 2C) shows a biphasic pulse 30 that has an asymmetric waveform. The biphasic pulse 30 comprises an anodic pulse 31 and a cathodic pulse 32 which both have a rectangular shape in the time-intensity diagram. The anodic pulse 31 is the stimulation pulse and the cathodic pulse 32 is the charge-compensation pulse. The area 33 covered by the anodic pulse 31 and the area 34 covered by the cathodic pulse 32 are identical such that the asymmetric biphasic pulse 30 is charged-balanced. However, pulse amplitude 35 and pulse duration 36 of the anodic pulse 31 are not identical to pulse amplitude 37 and pulse duration 38 of the cathodic pulse 32 leading to an asymmetric waveform. When further increasing the pulse duration 38 of the cathodic pulse 32 while keeping the waveform of the anodic pulse 31 fixed and maintaining the prerequisite of balanced charges, the waveform of the asymmetric biphasic pulse 30 tends towards a monophasic waveform such that the waveform of an asymmetric biphasic pulse is also referred to as pseudo-monophasic.

Figure 3 shows a train of pseudo-monophasic pulses comprising a first 40, a second 50 and a third 60 pseudo-monophasic pulse. Each of the three pulses comprises an anodic stimulation pulse and a cathodic charge-compensation pulse. Further, for each of the pseudo-monophasic pulses it holds that the area covered by the stimulation pulse and the area covered by the charge-compensation pulse of a respective pseudo-monophasic pulse have the same absolute value such that each of the pseudo-monophasic pulses is charge-balanced.

According to prior art, the ratio between pulse amplitude and pulse duration of a charge-compensation pulse of the first pseudo-monophasic pulse is fixed for each charge-compensation pulse of a respective pseudo-monophasic pulse within the train of pseudo-monophasic pulses, i.e. same fixed ratio is used in different pseudo-monophasic pulses. Further, the ratio is determined and limited by the largest pulse duration and the highest pulse amplitude of a stimulation pulse of the train of pseudo-monophasic pulses and the fastest stimulation rate within the train of pseudo-monophasic pulses. In the example shown, the largest pulse duration and the highest pulse amplitude of a stimulation pulse are given by the pulse duration 51 and the pulse amplitude 52 of the stimulation pulse 53 of the second pulse 50. The fastest stimulation rate is determined by the period duration 54 of the second pulse 50. Consequently, the ratio between pulse amplitude 55 and pulse duration 56 of the charge-compensation pulse 57 of the second pulse 50 determines the ratio used as a fixed ratio for the charge-compensation pulse 41 of the first pulse 40 and the charge-compensation pulse 61 of the third pulse 60.

The stimulation pulse 42 of the first pulse 40 and the stimulation pulse 62 of the third pulse 60 have shorter pulse duration and smaller pulse amplitude than the stimulation pulse 53 of the second pulse 50. Moreover, the stimulation rate 43 of the first pulse 40 is slower than the stimulation rate 54 of the second pulse 50. Or in other words, the current stimulation rate of the first pulse 40 is slower than the current stimulation rate 54 of the second pulse 50.

As a result of using a fixed ratio, the pulse duration of a charge-compensation pulse extends over the maximum time window available, only, for the second pseudo-monophasic pulse 50. At least for the first pulse 40, the maximum time window available for the duration of the charge-compensation pulse 41 is only partly used. In fact, the use of a fixed ratio between pulse amplitude and pulse duration of a charge-compensation pulse according to prior art leads to the consequence that at least for the first pulse 40 shown in the example, the waveform of the pseudo-monophasic pulse is not optimized in terms of its stimulation efficiency and thus could be improved.

Fig. 4 shows a train of optimized biphasic pulses (pseudo-monophasic pulses) according to an embodiment. The optimized biphasic pulses (pseudo-monophasic pulses) includes a first 70, a second 80 and a third 90 biphasic pulses. Each of the three pulses comprises an anodic stimulation pulse and a cathodic charge-compensation pulse. Further, for each of the pseudo-monophasic pulses it holds that the area covered by the stimulation pulse and the area covered by the charge-compensation pulse of a respective pseudo-monophasic pulse have the same absolute value such that each of the pseudo-monophasic pulses is charge-balanced.

Importantly, in the example shown, the ratio between pulse amplitude and pulse duration of the charge-compensation pulses within the train of pseudo-monophasic pulses is not fixed but variable. Due to the variable ratio, the pulse duration of each charge-compensation pulse can extend over the maximum time window available for each biphasic pulse.

Using the example of the first pulse 70, the maximum time window available for the pulse duration 78 of the charge-compensation pulse 74 is determined by subtracting the pulse duration 71 of the stimulation pulse 72, a delay 73 defined by a duration between an end time point of the stimulation pulse 72 and an onset time point of the charge-compensation pulse 74 and a delay 75 defined by a duration between an end time point of the charge-compensation pulse 74 and an onset time point of the stimulation pulse 81 of the consecutive second pulse 80 from the current period duration. The current period duration of the first pulse 70 is the inverse of the current stimulation rate 76 of the first pulse 70. The stimulation rate of the first pulse 70 is given i.e. by onset time point of the stimulation pulse 72 and the onset time point of the stimulation pulse 81. The maximum time window available can be calculated for each biphasic pulse individually within the train of biphasic pulses.

In view of the prerequisite of charge-balancing, not only the current stimulation rate corresponding to duration 76 of the first pulse 70 has to be considered but also the pulse amplitude 77 and pulse duration 71 of the respective stimulation pulse 72 as these define the charge to be compensated by the charge-compensation pulse 74. The charge to be compensated eventually defines the pulse amplitude 79 of the charge-compensation pulse 74.

As can be seen in Fig. 4, the maximum time window available for the charge-compensation pulse 82 of the second pulse 80 is smaller compared to the maximum time window available for the charge-compensation pulse 74 of the first pulse 80. This is mainly due to the fact, that the pulse duration 83 of the stimulation pulse 81 is longer than the pulse duration 71 of the stimulation pulse 72. Although the pulse amplitude 84 of stimulation pulse 81 and stimulation pulse 72 as well as the stimulation rates corresponding to durations 76 and 87 are identical, the ratio between pulse amplitude 85 and pulse duration 86 of the charge-compensation pulse 82 is significantly different to the ratio between pulse amplitude 79 and pulse duration 78 of the charge-compensation pulse 74. However, for both these biphasic pulses, the waveform is the most efficient for stimulating i.e. auditory nerve fibres as it is the waveform that is the closest to a pure monophasic waveform.

In the example shown, the stimulation rate across the biphasic pulses is illustrated as constant leading to a periodic stimulation rate of pseudo-monophasic pulses. In an alternative implementation of the example shown, a current stimulation rate varies leading to stimulation with an aperiodic stimulation rate. In another alternative implementation of the example shown, a first pulse of a pseudo-monophasic pulse is a cathodic charge-compensation pulse followed by an anodic stimulation pulse.

In another alternative implementation of the example shown, there is no delay such that the maximum time window available can be calculated by subtracting the pulse duration of a stimulation pulse from the current period duration. In another alternative implementation of the example shown, the amplitude of at least some stimulation pulses within a train of pseudo-monophasic pulses changes which can be considered for calculating the waveform of a respective charge-compensation pulse.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, elements, components, and/or steps but do not preclude the presence or addition of one or more other features, elements, components, and/or steps thereof. It will also be understood that when an element is referred to as being "connected" or "coupled" to another element, it can be directly connected or coupled to the other element but an intervening elements may also be present, unless expressly stated otherwise. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The scope should be judged in terms of the claims that follow.

## Claims

1. A hearing aid implant comprising
a control unit configured
to generate a train of biphasic electric pulses in dependence upon an electric audio signal, wherein each biphasic electric pulse comprises a stimulation pulse of a first polarity and a charge-compensation pulse of a second polarity opposite to said first polarity of the stimulation pulse, and
to i) adjust a current stimulation rate relating to a first stimulation pulse of said train of biphasic electric pulses to generate an adjusted stimulation rate relating to a second stimulation pulse of said train of biphasic electric pulses, and ii) adjust at least one of pulse amplitude or pulse duration relating to the first stimulation pulse and at least one of pulse amplitude or pulse duration relating to the second stimulation pulse,
a stimulation unit configured to deliver said train of biphasic electric pulses to an electrode array, said electrode array comprising a number of electrodes for providing electric stimulation with the first stimulation pulse at the current stimulation rate and/ or with the second stimulation pulse at the adjusted stimulation rate,
the control unit is further configured to
i) calculate a first pulse amplitude and first pulse duration of a first charge-compensation pulse corresponding to the first stimulation pulse such that the first charge-compensation pulse has the first pulse amplitude and first pulse duration that result in a charge compensation of the charge caused by the first stimulation pulse, and the first pulse duration is according to the current stimulation rate, and/ or
ii) calculate a second pulse amplitude and second pulse duration of a second charge-compensation pulse corresponding to the second stimulation pulse such that the charge-compensation pulse has the second pulse amplitude and second pulse duration that result in a charge compensation of the charge caused by the second stimulation pulse, and the second pulse duration is according to the adjusted stimulation rate.

2. The hearing aid implant according to claim 1, wherein
the first pulse duration corresponding to the current stimulation rate is at least a part of a first remaining time, which the control unit is configured to determine by calculating a difference between an inverse of the current stimulation rate and the pulse duration relating to the first stimulation pulse or between inverse of the current stimulation rate and the pulse duration relating to the first stimulation pulse and at least one of a delay defined by a duration between an end time point of the first stimulation pulse and an onset time point of the first charge-compensation pulse or a delay defined by a duration between an onset time point of a first biphasic pulse including the first stimulation pulse and an onset time point of the first stimulation pulse of the first biphasic pulse; and/ or
the second pulse duration corresponding to the adjusted stimulation rate is at least a part of a second remaining time, which the control unit is configured to determine by calculating a difference between inverse of the adjusted stimulation rate and the pulse duration relating to the second stimulation pulse or between inverse of the adjusted stimulation rate and the pulse duration relating to the second stimulation pulse and at least one of a delay defined by a duration between an end time point of the second stimulation pulse and an onset time point of the second charge-compensation pulse or a delay defined by a duration between an onset time point of a second biphasic pulse including the second stimulation pulse and an onset time point of the second stimulation pulse of the second biphasic pulse.

3. The hearing aid implant according to any of the preceding claims, wherein the control unit is configured to
i) maximize the first pulse duration by utilizing the first remaining time entirely as the first pulse duration and accordingly calculate the first pulse amplitude to charge compensate the charge caused by the first stimulation pulse; and/ or
ii) maximize the second pulse duration utilizing the second remaining time entirely as the second pulse duration and accordingly calculate the second pulse amplitude to charge compensate the charge caused by the second stimulation pulse.

4. The hearing aid implant according to any of the preceding claims, wherein the stimulation pulse and charge-compensation pulse for at least one of the electric biphasic pulse of the train of biphasic pulses are individually rectangular and asymmetric to each other, wherein
the control unit is configured to calculate a ratio specific to the stimulation rate of the at least one of the electric biphasic pulse and the asymmetry is defined by the calculated ratio.

5. The hearing aid implant according to any of the preceding claims, wherein the control unit is configured to vary a ratio between a pulse amplitude of a stimulation pulse and pulse amplitude of corresponding charge-compensation pulse across different biphasic pulses of the train of biphasic pulses.

6. The hearing aid according to any of the preceding claims, wherein the control unit is configured to vary a ratio between a pulse amplitude of the stimulation pulse and pulse amplitude of corresponding charge-compensation pulse of at least one biphasic electric pulse during hearing aid implant operation in accordance with a ratio between the pulse duration of the charge-compensation pulse and the pulse duration of the stimulation pulse of the at least one biphasic electric pulse.

7. The hearing aid implant according to any of the preceding claims, wherein the control unit is configured to
calculate a first ratio as a ratio between a pulse duration of a first stimulation pulse and first pulse duration of a first charge compensation pulse of a first biphasic pulse and calculate a second ratio as a ratio between a pulse duration of a second stimulation pulse and a second pulse duration of a second charge compensation pulse of a second biphasic pulse; and
change the first ratio for the first biphasic pulse comprising the first stimulation pulse and first charge-compensation pulse to the second ratio for the second biphasic pulse comprising the second stimulation pulse and second charge-compensation pulse.

8. A hearing aid implant according to any of the preceding claims, wherein the electrode array is configured to be positioned within a cochlea and/or within a modiolus and/ or at an auditory brainstem of a user of the hearing aid implant.

9. An implantable hearing aid system comprising the hearing aid implant of any of the preceding claims 1 to 8.

10. An implantable hearing aid system according to claim 9, wherein the hearing aid system is selected from a group consisting of cochlear implant, auditory brainstem implant and transmodiolar implant that comprises an electrode array configured to be positioned within the modiolus of a user.

11. A method of generating a biphasic electric pulse comprising
generating a train of biphasic electric pulses in dependence upon an electric audio signal, wherein each biphasic electric pulse comprises a stimulation pulse of a first polarity and a charge-compensation pulse of a second polarity that is opposite to said first polarity of the stimulation pulse,
adjusting a current stimulation rate relating to a first stimulation pulse of said train of biphasic electric pulses to generate an adjusted stimulation rate relating to a second stimulation pulse of said train of biphasic electric pulses and adjusting at least one of pulse amplitude or pulse duration relating to the first stimulation pulse and at least one of pulse amplitude or pulse duration relating to the second stimulation pulse;
delivering a first biphasic electric pulse and/ or a second biphasic electric pulse of the train of biphasic electric pulses to an electrode array comprising a number of electrodes, the first biphasic electric pulse comprising the first stimulation pulse and a first change-compensation electric pulse and the second biphasic electric pulse comprising the second stimulation pulse and a second charge compensation electric pulse;
calculating a first pulse amplitude and first pulse duration of the first charge-compensation pulse such that the first charge-compensation pulse has the first pulse amplitude and first pulse duration that result in a charge compensation of the charge caused by the first stimulation pulse, and the first pulse duration is according to the current stimulation rate, and/ or
calculating a second pulse amplitude and second pulse duration of the second charge-compensation pulse such that the charge-compensation pulse has the second pulse amplitude and second pulse duration that result in a charge compensation of the charge caused by the second stimulation pulse, and the second pulse duration is according to the adjusted stimulation rate.

12. The method according to claim 11, further comprising
calculating a difference between i) an inverse of the current stimulation rate, and ii) the pulse duration relating to the first stimulation pulse or the pulse duration relating to the first stimulation pulse and a delay, the delay being defined by a duration between an end time point of the first stimulation pulse and an onset time point of the first charge-compensation pulse, and determining the first pulse duration comprising at least a part of the calculate difference; and/ or
determining the second pulse duration by calculating a difference between i) an inverse of the adjusted stimulation rate, and ii) the pulse duration relating to the second stimulation pulse or the pulse duration relating to the second stimulation pulse and a delay, the delay being defined by a duration between an end time point of the second stimulation pulse and an onset time point of the second charge-compensation pulse, and determining the first pulse duration comprising at least a part of the calculate difference.

13. The method according to any of the preceding claims 11-12, further comprising
i) based on the determined first pulse duration, calculating the first pulse amplitude to charge compensate the charge caused by the first stimulation pulse; and/ or
ii) based on the determined second pulse duration, calculating the second pulse amplitude to charge compensate the charge caused by the second stimulation pulse.

14. The method according to any of the preceding claims 11 to 13, further comprising varying a ratio between a pulse amplitude of the stimulation pulse and pulse amplitude of corresponding charge-compensation pulse of at least one biphasic electric pulse during hearing aid implant operation in accordance with a ratio between the pulse duration of the charge-compensation pulse and the pulse duration of the stimulation pulse of the at least one biphasic electric pulse.

15. The method according to any of the preceding claims 11-14, further comprising
calculating a first ratio as a ratio between a pulse duration of a first stimulation pulse and first pulse duration of a first charge compensation pulse of a first biphasic pulse and calculating a second ratio as a ratio between a pulse duration of a second stimulation pulse and a second pulse duration of a second charge compensation pulse of a second biphasic pulse; and
changing the first ratio for the first biphasic pulse to the second ratio for the second biphasic pulse.
